# EUROPÄISCHE PATENTSCHRIFT

(11) **EP 1 606 067 B1**
(45) Veröffentlichungstag und Bekanntmachung des Hinweises auf die Patenterteilung: **30.08.2006**
(21) Anmeldenummer: 04719941.9
(22) Anmeldetag: 12.03.2004
(51) Int. Cl.: B21B 13/06, B21B 35/06

(54) **STAUCHWALZWERK FÜR DEN WARMBETRIEB**
SWAGE ROLLING MILL FOR HEATED OPERATION
CAGE REFOULEUSE POUR FONCTIONNEMENT A CHAUD

(30) Priorität: 22.03.2003 DE 10312940
(43) Veröffentlichungstag der Anmeldung: 21.12.2005
(73) Patentinhaber: SMS Demag Aktiengesellschaft, 40237 Düsseldorf (DE)
(72) Erfinder: HABERMANN, Andreas, 52428 Jülich (DE); ZIESER, Bernd, 57250 Netphen (DE); ARTEL, Gerhard, 57399 Kirchhundem (DE)
(74) Vertreter: Valentin, Ekkehard
(86) Internationale Anmeldenummer: PCT/EP2004/002573
(87) Internationale Veröffentlichungsnummer: WO 2004/082859

(56) Entgegenhaltungen:
- EP-B- 0 491 785
- US-A- 2 575 231
- PATENT ABSTRACTS OF JAPAN Bd. 0090, Nr. 03 (M-349), 9. Januar 1985 (1985-01-09) & JP 59 156501 A (SUMITOMO JUKIKAI KOGYO KK), 5. September 1984 (1984-09-05)

## Beschreibung

Die Erfindung betrifft ein Walzwerk, insbesondere ein Stauchwalzwerk für den Warmbetrieb, mit einem Paar mit ihren Mittelachsen senkrecht angeordneten, gegeneinander anstellbaren Walzen, die mittels Gelenkwellen an zumindest einen Drehantrieb angeschlossen sind.

Derartige Stauchwalzwerke werden in erheblichem Umfang als Vertikalwalz- oder Stauchgerüst mit einem oder mehreren Querhäuptern ausgeführt, auf denen die schweren Drehantriebe für die Walzen gelagert sind (DE-A- 1 602 177). Die Walzen können auch in horizontal querverschieblichen Kassetten gelagert sein (EP 0 491 785 B1). Die Kassette kann auch vertikal verschiebbar sein (EP 0 493 430 B1). Gemäß einem älteren Vorschlag (DE-A- 2 227 549) können die Walzen senkrecht zwischen den Drehantrieben nach oben herausgezogen werden.

Den bekannten Dreh- und Anstellantrieben von Stauchgerüsten haften die Flachteile an, dass diese Bauweise technisch aufwändig und nicht sehr zuverlässig ist. Aufgrund der hohen Anzahl von Spielabständen ineinander bewegter Antriebsteile tritt ein hoher Verschleiß auf und dadurch entsteht ein hoher Wartungsaufwand. Mit der bekannten Antriebsanordnung ist ferner eine mangelhafte Zugänglichkeit für Wartungsarbeiten verbunden. Weitere Nachteile bilden ein träges Anstellverhalten der Stauchwalzen, die hohe Massen aufweisen, eine hohe Reibung verursachen und ungünstige Hebelarme für die Anstellung bedingen.

Der Erfindung liegt die Aufgabe zugrunde, ein günstigeres Anstellverhalten bei geringeren bewegten Massen zu erzielen, die Reibung zu vermindern und bessere Hebelarmverhältnisse zu erreichen.

Die gestellte Aufgabe wird erfindungsgemäß dadurch gelöst, dass der Drehantrieb für die beiden Walzen unterhalb der Hüttenflur-Ebene ortsfest angeordnet und mit jeweils einem ortsfesten Getriebe jeweils mit der Gelenkwelle antriebsmäßig verbunden ist. Dadurch kann das Anstellen der Stauchwalzen leichter und schneller erfolgen, zudem die bewegten Massen geringer sind. Das Getriebe wird nicht mehr zusammen mit den Stauchwalzen verfahren, sondern ist auf dem Fundament oder Fundament-Bereichen verankert. Es entsteht durch das geringere, bewegte Gewicht weniger Reibung. Die Hebelarm-Relationen sind durch kürzere Verstellorgane auch günstiger. Es entsteht auch weniger Wartungsaufwand und die Wartung ist einfacher, weil die Zugänglichkeit auf der Hüttenflur-Ebene und auf dem Niveau des Fundaments einfacher ist. Alle Vorteile zusammen ergeben eine höhere Anlagenverfügbarkeit, eine höhere Betriebsbereitschaft und dadurch kann auch eine optimierte Produktqualität erzielt werden.

Die Verringerung der bewegten Massen kann nach einer Ausgestattung dadurch weiter entwickelt werden, dass der Drehantrieb mittels einer durchgehenden Antriebswelle und beidseitig abgezweigten Kegelradgetrieben bzw. jeweils mit einem Stirnradgetriebe an die jeweilige Gelenkwelle angeschlossen ist.

Weitergehend kann auch Gewicht dadurch örtlich festgelegt werden, dass die Anstellantriebe auf beiden Seiten der senkrechten Walzen über der Hüttenflur-Ebene angeordnet sind. Die Stauchwalzen können zwischen den Anstellantrieben frei nach oben beim Ausbau ausgehoben und beim Einbau eingefädelt werden.

Die im unteren Fundamentbereich liegenden Antriebskomponenten werden gemäß einer Weiterbildung dadurch geschützt, dass an den Gelenkwellenkopf-Aufnehmern der Gelenkwellen zusammen mit den Walzen verfahrbare Abweiser-Bleche befestigt sind.

Dabei wird ein Sammeln und Führen der abzuleitenden Verfahrens-Abfalistoffen dadurch geschaffen, dass die verfahrbaren Abweiser-Bleche einen im wesentlichen senkrechten ersten Schacht bilden.

Eine Weiterentwicklung dieses Systems besteht darin, dass zwischen den feststehenden Stirnradgetrieben der Gelenkwellen ein zweiter an den ersten Schacht anschließender Schacht mit feststehenden Abweiser-Blechen gebildet ist.

Der Schutz der tiefer liegenden Antriebskomponenten kann noch erhöht werden, indem die feststehenden Abweiser-Blechen einen an die verfahrbaren Abweiser-Bleche anschließenden und unmittelbar gegenüberliegenden trapez- oder konusförmigen Eingang bilden.

Die sich in dem ersten Schacht und dem zweiten Schacht ansammetndenVerfahrens-Abfallstoffe können derart weiterbefördert und entsorgt werden, dass unterhalb des zweiten Schachtes eine rinnenförmige Sammelgrube zum Abführen von Schmutz, Zunder, Schmutzwasser u. dgl. vorgesehen ist.

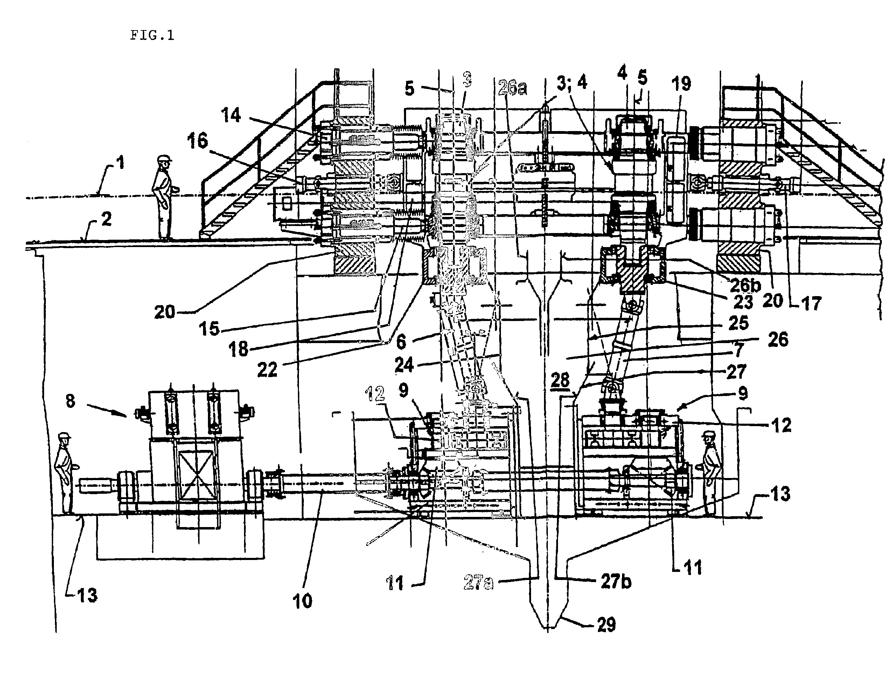

In der Zeichnung ist das Walzwerk dargestellt, das nachstehend näher erläutert wird.

Die einzige Figur der Zeichnung zeigt eine Vorderansicht des Walzwerks, in der sich das Walzgut senkrecht zur Zeichenebene bewegt.

Das im Querschnitt senkrecht zur Walzrichtung gezeigte Walzwerk ist als Stauchwalzwerk für den Warmbetrieb ausgeführt. Der unmittelbare Verformungsbereich 1 liegt über der Hüttenflur-Ebene 2. Die Walzen 3 und 4 stehen mit ihren Mittelachsen 5 senkrecht und sind mittels Gelenkwellen 6 und 7 an zumindest einen Drehantrieb 8 angeschlossen.

Das Besondere ist nunmehr, dass der Drehantrieb 8 für die beiden Walzen 3 und 4 unter der Hüttenflur-Ebene 2 ortsfest angeordnet und mit jeweils einem ortsfesten Getriebe 9 und jeweils auf beiden Seiten mit einer Gelenkwelle 6, 7 antriebsmäßig verbunden ist.

Zur Antriebsübertragung, ausgehend vom Drehantrieb 8 (der aus einem schweren elektrischen Motor besteht) wird die Antriebskraft mittels einer durchgehenden, drehgelagerten Antriebswelle 10 und beidseitig abgezweigten Kegelradgetriebe (-Stufen) 11 bzw. jeweils einem einstufigen Stirnradgetriebe 12 auf die jeweilige Gelenkwelle 6 und 7 übertragen.

Im Gegensatz zur Lagerung des Drehantriebs 8 unter der Hüttenflur-Ebene 2 auf einem tief angelegten Fundament 13 sind die Anstellantriebe 14 und 15 auf beiden Seiten der senkrechten Walzen 3, 4 über der Hüttenflur-Ebene 2 angeordnet.

Zwischen den beiderseitigen paarweisen Anstellantrieben 14 und 15 sind jeweils hydraulisch betätigte Kolben-Zylinder-Einheiten 16 und 17 für Walzentraversen 18, 19 in den Ständern 20 des Walzgerüstes befestigt.

An Gelenkwellenkopf-Aufnehmern 22 und 23 der Gelenkwellen 6, 7 sind Abweiser-Bleche 24 und 25 befestigt und werden bei Verstellung der Walzen 3, 4 mitbewegt. Das Paar der Abweiser-Bleche 24, 25 bildet einen ersten, senkrechten Schacht 26 oder zwei nebeneinanderliegende Teilschächte 26a und 26b.

Zwischen den feststehenden Stirnradgetrieben 12 der Gelenkwelle 6; 7 ist ein zweiter Schacht 27 gebildet, der aus feststehenden Abweiser-Blechen 27a, 27b hergestellt ist.

Die feststehenden Abweiser-Bleche 27a, 27b formen einen an die verfahrbaren Abweiser-Bleche 24, 25 anschließenden und unmittelbar gegenüberliegenden trapez- oder konusförmigen Eingang 28.

Unterhalb des zweiten Schachtes 27 ist eine rinnenförmige Sammelgrube 29 zum Abführen des gesammelten Schmutzes, Zunders, Schmutzwassers u. dgl. in das Fundament eingeformt.

### Bezugszeichenliste

- 1: unmittelbarer Verformungsbereich
- 2: Hüttenflur-Ebene
- 3: Walze
- 4: Wälze
- 5: Mittelachse
- 6: Gelenkwelle
- 7: Gelenkwelle
- 8: Drehantriebsmotor
- 9: ortsfestes Getriebe
- 10: Antriebswelle
- 11: Kegelradgetriebe
- 12: Stirnradgetriebe (-Stufe)
- 13: Fundament
- 14: Anstellantrieb
- 15: Anstellantrieb
- 16: Kolben-Zylinder-Einheit
- 17: Kolben-Zylinder-Einheit
- 18: Walzentraverse
- 19: Walzentraverse
- 20: Walzenständer
- 21:
- 22: Gelenkwellenkopf-Aufnehmer
- 23: Gelenkwellenkopf-Aufnehmer
- 24: bewegtes Abweiser-Blech
- 25: bewegtes Abweiser-Blech
- 26: erster Schacht
- 26a: Teilschacht
- 26b: Teilschacht
- 27: zweiter Schacht
- 27a: feststehendes Abweiser-Blech
- 27b: feststehendes Abweiser-Blech
- 27: trapez- oder konusförmiger Eingang
- 28: rinnenförmige Sammelgrube

## Patentansprüche

1. Stauchwalzwerk für den Warmbetrieb, mit einem Paar mit den Mittelachsen (5) senkrecht angeordneten, gegeneinander anstellbaren Walzen (3;4), die mittels Gelenkwellen (6;7) an einen Drehantrieb angeschlossen sind,
**dadurch gekennzeichnet,**
**dass** ein Drehantriebsmotor (8) für die beiden Walzen (3; 4) unterhalb der Hüttenflur-Ebene (2) ortsfest angeordnet und mit jeweils einem ortsfesten Getriebe (9) und dieses jeweils mit seiner Gelenkwelle (6; 7) antriebsmäßig verbunden ist.

2. Stauchwalzwerk nach Anspruch 1,
**dadurch gekennzeichnet,**
**dass** der Drehantriebsmotor (8) mittels einer durchgehenden Antriebswelle (10) und beidseitig abgezweigten Kegelradgetrieben (11) bzw. jeweils mit einem Stirnradgetriebe (12) an die jeweilige Gelenkwelle (6; 7) angeschlossen ist.

3. Stauchwalzwerk nach einem der Ansprüche 1 oder 2,
**dadurch gekennzeichnet,**
**dass** die Anstellantriebe (14; 15) auf beiden Seiten der senkrechten Walzen (3; 4) über der Hüttenflur-Ebene (2) angeordnet sind.

4. Stauchwalzwerk nach einem der Ansprüche 1 bis 3,
**dadurch gekennzeichnet,**
**dass** an den Gelenkwellenkopf-Aufnehmem (22; 23) der Gelenkwellen (6; 7) zusammen mit den Walzen (3; 4) verfahrbare Abweiser-Bleche (24; 25) befestigt sind.

5. Stauchwalzwerk nach Anspruch 4,
**dadurch gekennzeichnet,**
**dass** die verfahrbaren Abweiser-Bleche (24; 25) einen im wesentlichen senkrechten ersten Schacht (26) bilden.

6. Stauchwalzwerk nach den Ansprüchen 4 oder 5,
**dadurch gekennzeichnet,**
**dass** zwischen den feststehenden Stirnradgetrieben (12) der Gelenkwellen (6; 7) ein zweiter an den ersten Schacht (26) anschließender Schacht (27) mit feststehenden Abweiser-Blechen (27a; 27b) gebildet ist.

7. Stauchwalzwerk nach einem der Ansprüche 4 bis 6,
**dadurch gekennzeichnet,**
**dass** die feststehenden Abweiser-Bleche (27a; 27b) einen an die verfahrbaren Abweiser-Bleche (24; 25) anschließenden und unmittelbar gegenüberliegenden trapez- oder konusförmigen Eingang (28) bilden.

8. Stauchwalzwerk nach einem der Ansprüche 4 bis 7,
**dadurch gekennzeichnet,**
**dass** unterhalb des zweiten Schachtes (27) eine rinnenförmige Sammelgrube (29) zum Abführen von Schmutz, Zunder, Schmutzwasser u. dgl. vorgesehen ist.

## Claims

1. Upsetting rolling mill for hot operation, comprising a pair of mutually adjustable rolls (3; 4) which are arranged with the centre axes (5) vertical and which are connected with a rotary drive by means of universal joint shafts (6; 7), **characterised in that** a rotary drive motor (8) for the two rolls (3; 4) is arranged in stationary position below the plane (2) of the shop floor and is connected in each instance with a transmission (9), which is arranged in stationary location, and this is connected in terms of drive in each instance with its universal joint shaft (6; 7).

2. Upsetting rolling mill according to claim 1, **characterised in that** the rotary drive motor (8) is connected with the respective universal joint shaft (6; 7) by means of a continuous drive shaft (10) and bevel gear transmissions (11), which branch off at both sides, or by a respective spur gear transmission (12).

3. Upsetting rolling mill according to one of claims 1 and 2, **characterised in that** the adjusting drives (14; 15) on both sides of the vertical rolls (3; 4) are arranged above the plane (2) of the shop floor.

4. Upsetting rolling mill according to one of claims 1 to 3, **characterised in that** deflector plates (24; 25) are fastened to the universal joint shaft head sockets (22; 23) of the universal joint shafts (6; 7) to be movable together with the rolls (3; 4).

5. Upsetting rolling mill according to claim 4, **characterised in that** the movable deflector plates (24; 25) form a substantially vertical first well (26).

6. Upsetting rolling mill according to claim 4 or 5, **characterised in that** a second well (27), which adjoins the first well (26), with stationary deflector plates (27a; 27b) is formed between the stationary spur gear transmissions (12) of the universal joint shafts (6; 7).

7. Upsetting rolling mill according to one of claims 4 to 6, **characterised in that** the stationary deflector plates (27a; 27b) form a trapezium-shaped or conical inlet (28) adjoining the movable deflector plates (24; 25) and disposed directly opposite.

8. Upsetting rolling mill according to one of claims 4 to 7, **characterised in that** a channel-shaped collecting pit (29) for the discharge of dirt, cinder, contaminated water and the like is provided below the second well (27).

## Revendications

1. Laminoir de refoulement pour un fonctionnement à chaud avec une paire de cylindres (3,4) disposés verticalement avec leurs axes centraux (5), pouvant être réglés l'un contre l'autre, qui sont raccordés au moyen d'arbres articulés (6, 7) à un dispositif rotatif, **caractérisé en ce qu'**un moteur d'entraînement rotatif (8) pour les deux cylindres (3, 4) est disposé en un lieu fixe au-dessous du plan (2) du sol de l'usine métallurgique et est raccordé avec à chaque fois un dispositif d'entraînement fixe (9) et celui-ci est à chaque fois raccordé avec son arbre articulé (6, 7) dans le sens d'un entraînement.

2. Laminoir de refoulement selon la revendication 1, **caractérisé en ce que** le moteur d'entraînement rotatif (8) est raccordé au moyen d'un arbre d'entraînement (10) continu et deux commandes par engrenage conique (11) déviées des deux côtés ou à chaque fois avec un engrenage droit (12) à chaque arbre articulé (6, 7).

3. Laminoir de refoulement selon l'une quelconque des revendications 1 ou 2, **caractérisé en ce que** les dispositifs d'entraînement (14, 15) sont disposés des deux côtés des cylindres verticaux (3, 4) au-dessus du plan (2) du sol de l'usine métallurgique.

4. Laminoir de refoulement selon l'une quelconque des revendications 1 à 3, **caractérisé en ce qu'**on a fixé, sur les pièces (22, 23) qui reçoivent la tête des arbres articulés (6, 7) des tôles déflectrices (24, 25) pouvant être déplacées avec les cylindres (3, 4).

5. Laminoir de refoulement selon la revendication 4, **caractérisé en ce que** les tôles déflectrices (24, 25) forment un premier puits (26) essentiellement vertical.

6. Laminoir de refoulement selon l'une quelconque des revendications 4 ou 5, **caractérisé en ce qu'**on forme entre les engrenages droits fixes (12) des arbres articulés (6, 7) un deuxième puits (27) se raccordant au premier puits (26) avec des tôles déflectrices (27a, 27b) fixes.

7. Laminoir de refoulement selon l'une quelconque des revendications 4 à 6, **caractérisé en ce que** les tôles déflectrices fixes (27a, 27b) forment une entrée (28) trapézoïdale ou conique se raccordant aux tôles déflectrices (24, 25) mobiles et se trouvant directement en face de celles-ci.

8. Laminoir de refoulement selon l'une quelconque des revendications 4 à 7, **caractérisé en ce qu'**on a prévu, sous le deuxième puits (27) une fosse de collecte (29) en forme de rainure pour évacuer les salissures, la calamine, les eaux sales etc.
